# FASCICULE DE BREVET EUROPEEN

(11) **EP 0 992 234 B1**
(45) Date de publication et mention de la délivrance du brevet: **24.07.2002**
(21) Numéro de dépôt: 99401837.2
(22) Date de dépôt: 21.07.1999
(51) Int. Cl.: A61K 7/043

(54) **Vernis-à-ongles aqueux à haute viscosité**
Wässrige Hochviskose Nagellackformulierungen
High-viscosity aqueous nail polish compositions

(30) Priorité: 12.08.1998 FR 9810333
(43) Date de publication de la demande: 12.04.2000
(73) Titulaire: L'OREAL, 75008 Paris (FR)
(72) Inventeur: de la Poterie, Valérie, 77820 Le Chatelet-en Brie (FR); Bernard, Pascale, 94370 Sucy-en-Brie (FR)
(74) Mandataire: Brédeville, Odile Marie

(56) Documents cités:
- EP-A- 0 391 322
- EP-A- 0 418 469
- EP-A- 0 648 485
- EP-A- 0 676 451
- EP-A- 0 680 742
- DE-A- 4 311 039
- DE-A- 19 521 500
- US-A- 5 120 529
- US-A- 5 601 808
- US-A- 5 681 550

## Description

La présente invention a pour objet un vernis-à-ongles comprenant une dispersion aqueuse de polymère filmogène et un agent épaississant. Ce vernis peut être employé pour le maquillage des ongles ou bien encore pour le traitement des ongles.

Il est connu des demandes EP-A-143480, FR-A-2399238, et EP-A-648485 des compositions de vernis-à-ongles fluides comprenant une dispersion aqueuse de particules de polymère filmogène.

Dans les vernis à ongles à milieu aqueux, il est courant d'épaissir la phase aqueuse par des agents épaississants. Ces compositions épaissies permettent de faciliter la prise du produit hors de son conditionnement sans perte significative, de répartir le produit de façon régulière sur la zone à traiter ou bien encore de pouvoir utiliser le produit dans des quantités suffisantes pour obtenir l'effet cosmétique recherché.

Toutefois, ces compositions étant fluides, il est souvent nécessaire d'appliquer deux couches de produit pour obtenir un film couvrant parfaitement l'ongle, c'est-à dire un film qui ne permette pas de voir l'ongle à travers le film. Il est donc souhaitable de disposer de vernis-à-ongles présentant une bonne propriété de couvrance.

Par ailleurs, un vernis-à-ongles doit présenter également les qualités suivantes : facilité d'étalement du vernis sur l'ongle et obtention d'un film brillant, homogène, présentant de bonnes propriétés de maquillage, notamment d'aspect lisse.

Le but de la présente invention est de disposer d'une composition de vernis-à-ongles comprenant une dispersion aqueuse de particules de polymère filmogène s'étalant facilement sur l'ongle et conduisant, après application en une seule couche sur l'ongle, à un film parfaitement brillant, homogène, couvrant l'ongle et lisse.

La demanderesse a découvert qu'une telle composition pouvait être obtenue en ajustant la viscosité et en employant un agent épaississant particulier.

Ainsi, l'invention a pour objet une composition de vernis-à-ongles comprenant une dispersion aqueuse de particules de polymère filmogène, caractérisée par le fait que la composition comprend au moins un polyuréthane associatif en une quantité efficace pour que la composition ait une viscosité d'au moins 2 Pa.s, mesurée à 23°C, à la vitesse de rotation de 100 tours/min.

L'invention a aussi pour objet l'utilisation d'un polyuréthane associatif dans un vernis-à-ongles comprenant une dispersion aqueuse de particules de polymère filmogène et ayant une viscosité d'au moins 2 Pa.s, mesurée à 23°C, à la vitesse de rotation de 100 tours/min.

L'invention a encore pour objet l'utilisation d'un polyuréthane associatif dans une composition de vernis-à-ongles comprenant une dispersion aqueuse de particules de polymère filmogène, en une quantité efficace pour que la composition ait une viscosité d'au moins 2 Pa.s, mesurée à 23°C, à la vitesse de rotation de 100 tours/min.

L'invention a également pour objet l'utilisation d'une composition telle que définie précédemment pour l'obtention d'un film brillant et/ou homogène et/ou couvrant.

L'invention a encore pour objet l'utilisation d'une composition telle que définie précédemment comme vernis monocouche.

L'invention a aussi pour objet l'utilisation d'une composition telle que définie précédemment comme vernis pelliculable.

L'invention a pour objet un procédé de maquillage ou de traitement cosmétique et non thérapeutique des ongles consistant à appliquer sur les ongles une composition telle que définie précédemment.

Les inventeurs ont constaté que les polyuréthanes associatifs permettent d'obtenir les propriétés remarquables de vernis-à-ongles et que d'autres agents épaississants connus dans les vernis à consistance fluide ne permettaient pas d'obtenir ces propriétés rhéologiques ou cosmétiques souhaitées. Par exemple, les argiles comme la bentonite ou la montmorillonite ne conduisent pas à des viscosités supérieures à 2 Pa.s ; les polymères cellulosiques comme l'hydroxyéthylcellulose conduisent à la matification du film pour les viscosités recherchées ; les polymères épaississants de type acrylique, y compris les polymères acryliques associatifs, ne permettent pas un étalement satisfaisant pour les viscosités souhaitées.

Grâce au polyuréthane associatif et à la viscosité élevée de la composition, on obtient un vernis-à-ongle qui s'étale facilement sur l'ongle et qui permet d'obtenir, par application en une seule couche, un film présentant une bonne couvrance. De plus, le film est parfaitement homogène et lisse, sans traces de pinceau. Le film obtenu après séchage présente aussi une bonne brillance : la brillance, mesurée à l'aide d'un brillancemètre BYK-GARDNER à un angle de faisceau lumineux de 60 °, est supérieure ou égale à 80, et notamment va de 80 à 100.
Le vernis selon l'invention permet le dépôt d'un film épais sur l'ongle tout en étant appliqué en une seule couche. Aussi, le vernis selon l'invention est parfaitement utilisable comme vernis pelliculable.

La viscosité de la composition selon l'invention est d'au moins 2 Pa.s (2000 cps) et de préférence inférieure à 8 Pa.s car au delà, la composition n'est plus applicable à l'aide d'un pinceau. Avantageusement, la viscosité de la composition peut aller de 4 Pa.s (4000 cps) à 6 Pa.s (6000 cps).
La viscosité est mesurée à 23 °C, avec un viscosimètre BROOKFIELD DVII équipé du mobile n°3, à une vitesse de rotation de 100 tours/min, la mesure étant effectuée après 10 minutes de rotation (temps au bout duquel on observe une stabilisation de la viscosité et de la vitesse de rotation du mobile).

Les polyuréthannes associatifs sont des copolymères séquencés non ioniques comportant dans la chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

En particulier, ces polymères comportent au moins deux chaînes lipophiles hydrocarbonées, ayant de C₆ à C₃₀ atomes de carbone, séparées par une séquence hydrophile, les chaînes hydrocarbonées peuvent être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polymères peuvent être séquencés sous forme de tribloc ou multibloc. Les séquences hydrophobes peuvent donc être à chaque extrémité de la chaîne (par exemple : copolymère tribloc à séquence centrale hydrophile) ou réparties à la fois aux extrémités et dans la chaîne (copolymère multiséquencé par exemple). Les polymères peuvent être également en greffons ou en étoile.

De préférence, les polymères sont des copolymères triblocs dont la séquence hydrophile est une chaîne polyoxyéthylénée comportant de 50 à 1 000 groupements oxyéthylénés. En général les polyuréthannes associatifs comportent une liaison uréthane entre les séquences hydrophiles, d'où l'origine du nom.

Par extension figurent aussi parmi les polyuréthannes associatifs, des polymères dont les séquence hydrophiles sont liées par d'autres liaisons chimiques que la liaison uréthane aux séquences lipophiles.

A titre d'exemple, des polymères associatifs utilisables dans l'invention, on peut citer le polymère C₁₆-OE₁₂₀-C₁₆ vendu par la société HULS (sous le nom Sérad FX1100, molécule à fonction uréthanne et poids moléculaire moyen en poids de 1300), OE étant un motif oxyéthyléné. Comme polymère associatif, on peut aussi utiliser aussi le Rhéolate 205 à fonction urée vendu par la société RHEOX ou encore le Rhéolate 208 ou 204. Ces polyuréthannes associatifs sont vendus sous forme pure.

Le produit DW 1206B de chez RHOM & HAAS à chaîne alkyle en C₂₀ et à liaison uréthane, vendu à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le Sérad FX1010, le Sérad FX1035 et le Serad 1070 vendus par la société HULS, le Rhéolate 255, le Rhéolate 278 et le Rhéolate 244 vendus par la société RHEOX. On peut aussi utiliser le produit DW 1206F et le DW 1206J, ainsi que l'Acrysol RM 184 ou l'Acrysol 44 de la société RHOM & HAAS, ou bien encore le Borchigel LW 44 de la société BORCHERS.

Les polymères utilisables dans l'invention sont en particulier ceux décrits dans l'article de G. Fonnum, J. Bakke et Fk. Hansen - Colloid Polym. Sci 271, 380.389 (1993).

La composition selon l'invention contient un ou plusieurs polyuréthanes associatifs en une quantité suffisante pour obtenir une composition ayant la viscosité indiquée précédemment. Avantageusement, le polyuréthane associatif peut être présent en une quantité allant de 0,5 à 5 % en poids par rapport au poids total de la composition.

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats). Les polymères filmogènes de type radicalaires peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

On utilise de préférence des polymères filmogènes radicalaires anioniques, c'est-à-dire des polymères ayant au moins un monomère à groupement acide.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C₁-C₂₀, de préférence en C₁-C₈, des (méth)acrylates d'aryle, en particulier d'aryle en C₆-C₁₀, des (méth)acrylates d'hydroxyalkyle, en particulier d'hydroxyalkyle en C₂-C₆.
Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle.
Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.
Parmi les (méth)acrylates d'aryle, on peut citer l'acrylate de benzyle et l'acrylate de phényle.
Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C₂-C₁₂. Parmi les N-alkyl (méth)acrylamides, on peut citer le N-éthyl acrylamide, le N-t-butyl acrylamide et le N-t-octyl acrylamide.

Les polymères vinyliques filmogènes peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.
Comme exemple d'esters vinyliques, on peut citer l'acétate de vinyle, le néodécanoate de vinyle, le pivalate de vinyle, le benzoate de vinyle et le t-butyl benzoate de vinyle.
Comme monomères styrèniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme polymère acrylique filmogène utilisable selon l'invention, on peut citer ceux vendus sous les dénominations NEOCRYL XK-90®, NEOCRYL A-1070®, NEOCRYL A-1090®, NEOCRYL BT-62®, NEOCRYL A-1079®, NEOCRYL A-523® par la société ZENECA, DOW LATEX 432® par la société DOW CHEMICAL.

On peut ainsi citer, parmi les polycondensats utilisables comme polymères filmogènes, les polyuréthannes anioniques, cationiques, non-ioniques ou amphotères, les polyuréthannes-acryliques, les polyuréthannes-polyvinylpirrolidones, les polyester-polyuréthannes, les polyéther-polyuréthannes, les polyurées, les polyurée/polyuréthannes, et leurs mélanges.
Le polyuréthanne peut être, par exemple, un copolymère polyuréthanne, polyurée/uréthanne, ou polyurée, aliphatique, cycloaliphatique ou aromatique, comportant seule ou en mélange :
- au moins une séquence d'origine polyester aliphatique et/ou cycloaliphatique et/ou aromatique, et/ou,
- au moins une séquence siliconée, ramifiée ou non, par exemple polydiméthylsiloxane ou polyméthylphénylsiloxane, et/ou
- au moins une séquence comportant des groupes fluorés.

Les polyuréthannes filmogènes tels que définis dans l'invention peuvent être également obtenus à partir de polyesters, ramifiés ou non, ou d'alkydes comportant des hydrogènes mobiles que l'on modifie par réaction avec un diisocyanate et un composé organique bifonctionnel (par exemple dihydro, diamino ou hydroxyamino), comportant en plus soit un groupement acide carboxylique ou carboxylate, soit un groupement acide sulfonique ou sulfonate, soit encore un groupement amine tertiaire neutralisable ou un groupement ammonium quaternaire.

Le polyuréthane filmogène peut être choisi parmi les polyester-polyuréthanes et les polyéther-polyuréthanes, et de préférence parmi les polyester-polyuréthanes anioniques et les polyéther-polyuréthanes anioniques.

Avantageusement, on peut utiliser comme dispersion aqueuse de particules de polymère filmogène une dispersion aqueuse de polyester polyuréthane anionique dont la taille des particules de polyuréthane va de 2 à 100 nm et/ou dont la dureté d'un film obtenu après séchage, durant 24 heures à 30 °C et à 50 % d'humidité relative, d'une couche de 300 µm d'épaisseur (avant séchage) d'une dispersion aqueuse à 28 % de matière sèche desdites particules de polyuréthane va de 50 à 300 secondes.
La dureté du film est mesurée selon la norme ASTM D-43-66, ou la norme NF-T 30-016 (octobre 1981), à l'aide d'un pendule de Persoz.
Ces dispersions aqueuses conviennent parfaitement pour l'obtention d'un vernis pelliculable.
De telles dispersions aqueuses de polyester-polyuréthane anionique sont notamment commercialisées sous les dénominations "AVALURE UR 405®, "AVALURE UR 410®", "SANCURE 2060®" par la société GOODRICH.

On peut également utiliser comme dispersion aqueuse de particules de polymère filmogène des dispersions aqueuses de polyéther-polyuréthane anionique telles que celles vendues sous les dénominations "SANCURE 878®" par la société GOODRICH, "NEOREZ R 970®" par la société ICI.

Parmi les polycondensats utilisables comme polymère filmogène, on peut également citer les polyesters, les polyesters amides, les polyesters à chaîne grasse, les polyamides, et les résines époxyesters.

Les polyesters peuvent être obtenus, de façon connue, par polycondensation d'acides dicarboxyliques avec des polyols, notamment des diols.
L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexa-nedicarboxylique, l'acide isophtalique, l'acide téréphtalique, l'acide 2,5-norborane dicarboxylique, l'acide diglycolique, l'acide thiodipropionique, l'acide 2,5-naphtalènedicarboxylique, l'acide 2,6-naphta-lènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement -SO₃M, avec M représentant un atome d'hydrogène, un ion ammonium NH₄⁺ ou un ion métallique, comme par exemple un ion Na⁺, Li⁺, K⁺, Mg²⁺, Ca²⁺, Cu²⁺, Fe²⁺, Fe³⁺. On peut utiliser notamment un monomère aromatique bifonctionnel comportant un tel groupement -SO₃M.

Le noyau aromatique du monomère aromatique bifonctionnel portant en outre un groupement -SO₃M tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement -SO₃M : l'acide sulfoisophtalique, l'acide sulfotéréphtalique, l'acide sulfophtalique, l'acide 4-sulfonaphta(ène-2,7-dicarboxylique.
On préfère utiliser dans les compositions objet de l'invention des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique. De tels polymères sont vendus par exemple sous le nom de marque Eastman AQ par la société Eastman Chemical Products.

Les polymères d'origine naturelle, éventuellement modifiés, peuvent être choisis parmi la résine shellac, la gomme de sandaraque, les dammars, les élémis, les copals, les polymères cellulosiques insolubles dans l'eau, et leurs mélanges.

On peut encore citer les polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthannes, les polyurées, les polyesters, les polyesteramides et/ou les alkydes. Ces polymères sont généralement appelés polymères hybrides.

La dispersion comprenant un ou plusieurs polymères filmogènes peut être préparée par l'homme du métier sur base de ses connaissances générales.

La taille des particules de polymères en dispersion aqueuse peut aller de 10 à 500 nm, et est de préférence de 20 à 300 nm.

Le polymère en dispersion aqueuse peut être présent dans la composition selon l'invention en une teneur allant de 1 % à 50 % en poids, de préférence de 5 % à 45% en poids de matière sèche de polymères filmogènes par rapport au poids total de la composition.

Pour améliorer les propriétés filmogènes de la composition selon l'invention, la composition peut contenir des agents plastifiants et/ou des agents de coalescence qui sont bien connus de l'homme du métier.

Par ailleurs, la composition selon l'invention peut contenir des adjuvants couramment utilisés dans les compositions cosmétiques, notamment topiques. On peut citer à titre d'exemple d'adjuvants les colorants, les pigments, les nacres, les laques, les agents anti-UV, les conservateurs, les tensioactifs, les agents d'étalement, les parfums, les agents hydratants. Bien entendu, l'homme du métier veillera à choisir ce ou ces éventuels adjuvants, et/ou leur quantité, de telle manière que les propriétés avantageuses de la composition selon l'invention ne soient pas, ou substantiellement pas, altérées par l'adjonction envisagée, et qui permette donc d'obtenir une composition avec la viscosité indiquée précédemment.

La composition selon l'invention peut être appliquée sur des ongles naturels ou artificiels.

On va maintenant donner des exemples illustrant la présente invention sans toutefois la limiter.

Pour chaque composition, la viscosité a été mesurée à 23 °C, à l'aide du viscosimètre BROOKFIELD DVII équipé du mobile n°3, à la vitesse de rotation de 100 tours/min ; la mesure a été effectuée après 10 min de rotation.

### Exemple 1 :

On a préparé un vernis-à-ongles, par simple mélange à température ambiante, ayant la composition suivante :
- Dispersion aqueuse de polyester-polyuréthane anionique à 35 % de matière sèche (AVALURE UR 405 de GOODRICH) 47 g
- Dispersion aqueuse de copolymère acrylate à 44 % de matière sèche 15 g
- Polyuréthane associatif en solution aqueuse à 44 % de matière active (BORCHIGEL LW 44 de BORCHERS) 1,5 g
- Pigments 1 g
- Plastifiant 2 g
- Eau qs 100 g

La composition a une viscosité de 4,5 Pa.s.
On a constaté que le vernis s'applique facilement sur l'ongle et permet d'obtenir, après l'application en une seule couche, un film lisse, homogène et brillant, ne présentant pas de trace de pinceau et couvrant parfaitement l'ongle.

### Exemple 2 :

On a préparé un vernis-à-ongles ayant la composition suivante :
- Dispersion aqueuse de polyester-polyuréthane anionique à 35 % de matière sèche (AVALURE UR 405 de GOODRICH) 45 g
- Dispersion aqueuse de copolymère acrylate à 44 % de matière sèche 25 g
- Polyuréthane associatif (Serad FX1100 de SERVO HÜLS) 0,7 g
- Pigments 1 g
- Plastifiant 2 g
- Eau qs 100 g

La composition a une viscosité de 5,2 Pa.s.
On a constaté que le vernis s'applique facilement sur l'ongle et permet d'obtenir, après l'application en une seule couche, un film lisse, homogène et brillant, ne présentant pas de trace de pinceau et couvrant parfaitement l'ongle.

### Exemples 3 à 8 : comparatifs

On a préparé une base filmogène ayant la composition suivante :

### Base :

- Dispersion aqueuse de polyester-polyuréthane anionique à 35 % de matière sèche (AVALURE UR 405 de GOODRICH) 62,1 g
- Dispersion aqueuse de copolymère acrylate à 44 % de matière sèche 19,9 g
- Ethanol 4,1 g
- Agents plastifiants 4,8 g
- Agent de coalescence 1,8
- Agent d'étalement 0,4 g
- Conservateurs qs
- Pigments 1 g
- Pâte pigmentaire 2 g
- Eau qs 100 g

On a ensuite préparé 6 vernis-à-ongles ayant les compositions données dans le tableau ci-après (base + épaississant) puis on a étudié les qualités de maquillage de ces vernis déposés en une couche: on a mesuré la brillance du film obtenu après séchage et on a évalué l'aspect du film et du maquillage.

On a obtenu les résultats suivants :

| **Exemple** | **3** | **4** | **5** | **6** | **7 (HI)** | **8 (HI)** |
|---|---|---|---|---|---|---|
| **Base** | 96,1 g | 92,9 g | 97,3 g | 97,2 g | 96,1 g | 96,6 g |
| **Epaississant** | 0,8 g E1 | 2 g E2 | 0,6 g E3 | 0,7 g E4 | 0,5 g E5 | 0,4 g E6 |
| **Viscosité (Pa.s)** | 4,01 | 3,76 | 4,43 | 4,54 | 3,68 | 3,73 |
| **Brillance** | 89 | 87 | 87 | 88 | 74 | 14 |
| **Aspect du film** | Lisse | Lisse | Lisse | Lisse | Pas très lisse | Inhomogène |
| **Maquillage de l'ongle** | Correct | Correct | Correct | Correct | Trace de pinceau | Inhomogène |
| HI : hors invention | | | | | | |

Agents épaississants : la teneur est donnée en matière active
E1 : Borchigel LW 44 de BORCHERS
E2 : Serad 1070 de SERVO HÜLS
E3 : Rhéolate 205 de RHEOX
E4 : Serad FX 1100 de SERVO HÜLS
E5 : Copolymère acrylique Acrysol 33 de ROHM & HAAS
E6 : Copolymère acrylique Thixol 53L de COATEX

La brillance a été mesurée pour un film humide déposé de 300 µm d'épaisseur déposé sur carte de contraste puis conservé pendant 24 h, dans une atmosphère à 50 % d'humidité relative, pendant 24 h. La mesure a été effectuée à l'aide d'un brillancemètre BYK-GARDNER avec un angle de faisceau lumineux de 60°.

On a ainsi constaté que seules les compositions 3 à 6 selon l'invention permettent d'obtenir un film brillant, lisse et ne présentant pas de trace de pinceau.
La composition 7 (hors invention) conduit à la formation d'un film moins brillant, pas très lisse, laissant apparaître des traces de pinceau.
La composition 8 (hors invention) conduit à la formation d'un film inhomogène et le maquillage n'est pas satisfaisant.

## Revendications

1. Composition de vernis-à-ongles comprenant une dispersion aqueuse de particules de polymère filmogène, **caractérisée par le fait que** la composition comprend au moins un polyuréthane associatif en une quantité efficace pour que la composition ait une viscosité d'au moins 2 Pa.s, mesurée à 23°C, à la vitesse de rotation de 100 tours/min.

2. Composition selon la revendication 1; **caractérisée par le fait que** la viscosité est inférieure à 8 Pa.s.

3. Composition cosmétique selon la revendication 1, **caractérisée par le fait que** la composition a une viscosité allant de 4 Pa.s à 6 Pa.s.

4. Composition selon l'une quelconque des revendications précédentes, **caractérisée par le fait que** le polyuréthanne associatif est un polymère séquencé ou greffé comportant au moins deux chaînes alkyles ayant de C₆ à C₃₀ d'atomes de carbone, séparées par une séquence hydrophile.

5. Composition selon l'une des revendications précédentes, dans laquelle le polyuréthanne associatif comporte une séquence hydrophile polyoxyéthylénée.

6. Composition selon l'une des revendications précédentes, dans laquelle le polyuréthanne associatif est un polymère tribloc.

7. Composition selon l'une quelconque des revendications précédentes, **caractérisée par le fait que** le polyuréthane est présent en une teneur allant de 0,5 à 5 % en poids par rapport au poids total de la composition.

8. Composition selon l'une quelconque des revendications précédentes, **caractérisé par le fait que** le polymère filmogène est choisi dans le groupe formé par les polymères radicalaires, les polycondensats, les polymères d'origine naturelle et leurs mélanges.

9. Composition selon la revendication 8, **caractérisé par le fait que** les polymères radicalaires sont choisis dans le groupe formé par les polymères vinyliques résultant de la polymérisation de monomères choisis parmi les acides carboxyliques insaturés α,β-éthyléniques, les esters de cesdits acides, les amides de cesdits acides, les esters vinyliques, les monomères styréniques.

10. Composition selon la revendication 9, **caractérisé par le fait que** les esters des acides carboxyliques insaturés α,β-éthyléniques sont choisis parmi les (méth)acrylates.

11. Composition selon la revendication 8, **caractérisé par le fait que** les polycondensats sont choisis dans le groupe formé par les polyuréthanes, les polyesters, les polesters amides, les polyamides, les résines époxyesters.

12. Composition selon la revendication 8, **caractérisé par le fait que** les polymères d'origine naturelle sont choisis dans le groupe formé par la résine shellac, la gomme de sandaraque, les dammars, les élémis, les copals, les polymères cellulosiques insolubles dans l'eau, et leurs mélanges.

13. Composition selon l'une quelconque des revendications 1 à 7, **caractérisée par le fait que** la dispersion aqueuse de polymère filmogène est une dispersion aqueuse de polyester polyuréthane anionique dont la taille des particules de polyuréthane va de 2 à 100 nm.

14. Composition selon l'une quelconque des revendications précédentes, **caractérisée par le fait que** la dispersion aqueuse de polymère filmogène est une dispersion aqueuse de polyester polyuréthane anionique dont la dureté d'un film obtenu après séchage, durant 24 heures à 30 °C et à 50 % d'humidité relative, d'une couche de 300 µm d'épaisseur d'une dispersion aqueuse à 28 % de matière sèche desdites particules de polyuréthane va de 50 à 300 secondes.

15. Composition selon l'une quelconque des revendications précédentes, **caractérisée par le fait que** le polymère filmogène est présent en une teneur allant de 1 % à 50 % en poids par rapport au poids total de la composition.

16. Composition selon l'une quelconque des revendications précédentes, **caractérisée par le fait que** le film obtenu après séchage a une brillance d'au moins 80.

17. Composition cosmétique selon l'une quelconque des revendications précédentes, **caractérisée par le fait qu'**elle comprend en outre au moins un adjuvant choisi dans le groupe formé par les agents plastifiants, les agents de coalescence, les colorants, les pigments, les nacres, les laques, les agents anti-UV, les conservateurs, les tensioactifs, les agents d'étalement, les parfums, les agents hydratants.

18. Utilisation d'un polyuréthane associatif dans un vernis-à-ongles ayant une viscosité d'au moins 2 Pa.s, mesurée à 23°C, à la vitesse de rotation de 100 tours/min, comprenant une dispersion aqueuse de particules de polymère filmogène.

19. Utilisation d'un polyuréthane associatif dans une composition de vernis-à-ongles comprenant une dispersion aqueuse de particules de polymère filmogène, en une quantité efficace pour que la composition ait une viscosité d'au moins 2 Pa.s, mesurée à 23°C, à la vitesse de rotation de 100 tours/min.

20. Utilisation d'une composition selon l'une quelconque des revendications 1 à 17 pour l'obtention d'un film brillant et/ou homogène et/ou couvrant.

21. Utilisation d'une composition selon l'une quelconque des revendications 1 à 17 comme vernis monocouche.

22. Utilisation d'une composition selon l'une quelconque des revendications 1 à 17 comme vernis pelliculable.

23. Procédé de maquillage ou de traitement cosmétique des ongles, **caractérisé par le fait que** l'on applique sur les ongles une composition selon l'une quelconque des revendications 1 à 17.

24. Procédé selon la revendication 23, **caractérisé par le fait que** l'on applique une couche de la composition.

## Patentansprüche

1. Zusammensetzung für Nagellack, die eine wässerige Dispersion von Partikeln eines filmbildenden Polymers enthält, **dadurch gekennzeichnet, dass** die Zusammensetzung mindestens ein assoziatives Polyurethan in einer Menge enthält, die bewirkt, dass die Zusammensetzung eine bei 23 °C und bei einer Rotationsgeschwindigkeit von 100 U/min bestimmte Viskosität von mindestens 2 Pa·s aufweist.

2. Zusammensetzung nach Anspruch 1, **dadurch gekennzeichnet, dass** die Viskosität unter 8 Pa.s beträgt.

3. Kosmetische Zusammensetzung nach Anspruch 1, **dadurch gekennzeichnet, dass** die Zusammensetzung eine Viskosität im Bereich von 4 Pa·s bis 6 Pa.s aufweist.

4. Zusammensetzung nach einem der vorhergehenden Ansprüche, **dadurch gekennzeichnet, dass** das assoziative Polyurethan ein sequenzielles Polymer oder ein Pfropfpolymer ist, das mindestens zwei Alkylketten mit 6 bis 30 Kohlenstoffatomen aufweist, die durch eine hydrophile Sequenz voneinander getrennt sind.

5. Zusammensetzung nach einem der vorhergehenden Ansprüche, worin das assoziative Polyurethan eine polyethoxylierte hydrophile Sequenz aufweist.

6. Zusammensetzung nach einem der vorhergehenden Ansprüche, worin das assoziative Polyurethan ein Triblock-Polymer ist.

7. Zusammensetzung nach einem der vorhergehenden Ansprüche, **dadurch gekennzeichnet, dass** das Polyurethan in einem Mengenanteil im Bereich von 0,5 bis 5 Gew.-%, bezogen auf das Gesamtgewicht der Zusammensetzung, vorliegt.

8. Zusammensetzung nach einem der vorhergehenden Ansprüche, **dadurch gekennzeichnet, dass** das filmbildende Polymer unter durch radikalische Polymerisation hergestellten Polymeren, Polykondensaten, Polymeren natürlichen Ursprungs und deren Gemischen ausgewählt ist.

9. Zusammensetzung nach Anspruch 8, **dadurch gekennzeichnet, dass** die durch radikalische Polymerisation hergestellten Polymere unter Vinylpolymeren ausgewählt sind, die das Ergebnis der Polymerisation von Monomeren sind, die unter α,β-ethylenisch ungesättigten Carbonsäuren, den Estern dieser Säuren, den Amiden dieser Säuren, Vinylestern und Styrolmonomeren ausgewählt sind.

10. Zusammensetzung nach Anspruch 9, **dadurch gekennzeichnet, dass** die Ester der α,β-ethylenisch ungesättigten Carbonsäuren unter (Meth)acrylaten ausgewählt sind.

11. Zusammensetzung nach Anspruch 8, **dadurch gekennzeichnet, dass** die Polykondensate unter Polyurethanen, Polyestern, Polyesteramiden, Polyamiden und Epoxyesterharzen ausgewählt sind.

12. Zusammensetzung nach Anspruch 8, **dadurch gekennzeichnet, dass** die Polymere natürlichen Ursprungs unter Schellackharz, Sandarak, Dammarharzen, Elemiharzen, Kopalen, in Wasser unlöslichen Cellulosepolymeren und den Gemischen dieser Polymere ausgewählt sind.

13. Zusammensetzung nach einem der Ansprüche 1 bis 7, **dadurch gekennzeichnet, dass** die wässerige Dispersion eines filmbildenden Polymers eine wässerige Dispersion eines anionischen Polyester-Polyurethans ist, wobei die Größe der Polyurethan-Partikel im Bereich von 2 bis 100 nm liegt.

14. Zusammensetzung nach einem der vorhergehenden Ansprüche, **dadurch gekennzeichnet, dass** die wässerige Dispersion eines filmbildenden Polymers eine wässerige Dispersion eines anionischen Polyester-Polyurethans ist, wobei die Härte eines Films, der nach dem Trocknen einer 300 µm dicken Schicht einer wässerigen Dispersion dieser Polyurethan-Partikel mit einem Trockensubstanzgehalt von 28 % während einer Zeitspanne von 24 Stunden bei 30 °C und einer relativen Feuchtigkeit von 50 % erhalten wird, im Bereich von 50 bis 300 Sekunden liegt.

15. Zusammensetzung nach einem der vorhergehenden Ansprüche, **dadurch gekennzeichnet, dass** das filmbildende Polymer in einem Mengenanteil im Bereich von 1 bis 50 Gew.-%, bezogen auf das Gesamtgewicht der Zusammensetzung, vorliegt.

16. Zusammensetzung nach einem der vorhergehenden Ansprüche, **dadurch gekennzeichnet, dass** der nach dem Trocknen erhaltene Film einen Glanz von mindestens 80 aufweist.

17. Kosmetische Zusammensetzung nach einem der vorhergehenden Ansprüche, **dadurch gekennzeichnet, dass** sie ferner mindestens einen Zusatzstoff enthält, der unter Weichmachern, Koaleszenzmitteln, Farbstoffen, Pigmenten, Perlglanzmitteln, Lacken, Wirkstoffen gegen UV-Strahlung, Konservierungsstoffen, grenzflächenaktiven Stoffen, Mitteln zur Verteilung, Parfums und Hydratisierungsmitteln ausgewählt ist.

18. Verwendung eines assoziativen Polyurethans in einem Nagellack, der eine bei 23 °C und einer Rotationsgeschwindigkeit von 100 U/min bestimmte Viskosität von mindestens 2 Pa.s aufweist und der eine wässerige Dispersion von Partikeln eines filmbildenden Polymers enthält.

19. Verwendung eines assoziativen Polyurethans in einer Zusammensetzung für Nagellack, die eine wässerige Dispersion von Partikeln eines filmbildenden Polymers enthält, in einer Menge, die bewirkt, dass die Zusammensetzung eine bei 23 °C und einer Rotationsgeschwindigkeit von 100 U/min bestimmte Viskosität von mindestens 2 Pa·s aufweist.

20. Verwendung einer Zusammensetzung nach einem der Ansprüche 1 bis 17 zur Herstellung eines glänzenden und/oder homogenen und/oder deckenden Films.

21. Verwendung einer Zusammensetzung nach einem der Ansprüche 1 bis 17 als einschichtiger Lack.

22. Verwendung einer Zusammensetzung nach einem der Ansprüche 1 bis 17 als ablösbarer Lack.

23. Verfahren zum Schminken oder zur kosmetischen Behandlung der Nägel, **dadurch gekennzeichnet, dass** eine Zusammensetzung nach einem der Ansprüche 1 bis 17 auf die Nägel aufgetragen wird.

24. Verfahren nach Anspruch 23, **dadurch gekennzeichnet, dass** eine Schicht der Zusammensetzung aufgetragen wird.

## Claims

1. Nail varnish composition comprising an aqueous dispersion of film-forming polymer particles, **characterized in that** the composition comprises at least one associative polyurethane in an effective quantity such that the composition has a viscosity of at least 2 Pa.s., measured at 23°C at a rotation rate of 100 rpm.

2. Composition according to Claim 1, **characterized in that** the viscosity is less than 8 Pa.s.

3. Cosmetic composition according to Claim 1, **characterized in that** the viscosity of the composition is in the range 4 Pa.s. to 6 Pa.s.

4. Composition according to any one of the preceding claims, **characterized in that** the associative polyurethane is a block or graft polymer comprising at least two alkyl chains containing C₆ to C₃₀ carbon atoms, separated by a hydrophilic sequence.

5. Composition according to one of the preceding claims, in which the associative polyurethane comprises a polyoxyethylenated hydrophilic sequence.

6. Composition according to one of the preceding claims, in which the associative polyurethane is a triblock polymer.

7. Composition according to any one of the preceding claims, **characterized in that** the polyurethane is present in a quantity in the range from 0.5% to 5% by weight with respect to the total composition weight.

8. Composition according to any one of the preceding claims, **characterized in that** the film-forming polymer is selected from the group formed by free-radical polymers, polycondensates, polymers of natural origin and mixtures thereof.

9. Composition according to Claim 8, **characterized in that** the free-radical polymers are selected from the group formed by vinyl polymers resulting from the polymerization of monomers selected from unsaturated α,β-ethylenic carboxylic acids, esters of said acids, amides of said acids, vinyl esters and styrene monomers.

10. Composition according to Claim 9, **characterized in that** the esters of the unsaturated α,β-ethylenic carboxylic acids are selected from (meth)acrylates.

11. Composition according to Claim 8, **characterized in that** the polycondensates are selected from the group formed by polyurethanes, polyesters, polyesteramides, polyamides and epoxyester resins.

12. Composition according to Claim 8, **characterized in that** the polymers of natural origin are selected from the group formed by shellac resin, sandarac gum, dammar resins, elemi resins, copal resins, water-insoluble cellulosic polymers and mixtures thereof.

13. Composition according to any one of Claims 1 to 7, **characterized in that** the aqueous film-forming polymer dispersion is an aqueous dispersion of anionic polyester-polyurethane with a polyurethane particle size in the range from 2 nm to 100 nm.

14. Composition according to any one of the preceding claims, **characterized in that** the aqueous film-forming polymer dispersion is an aqueous anionic polyester-polyurethane dispersion wherein the hardness of a film obtained after drying a 300 µm thick coat of an aqueous dispersion containing 28% dry matter of said polyurethane particles for 24 hours at 30°C and at 50% relative humidity is in the range from 50 seconds to 300 seconds.

15. Composition according to any one of the preceding claims, **characterized in that** the film-forming polymer is present in a quantity in the range from 1% to 50% by weight with respect to the total composition weight.

16. Composition according to any one of the preceding claims, **characterized in that** the gloss of the film obtained after drying is at least 80.

17. Cosmetic composition according to any one of the preceding claims, **characterized in that** it further comprises at least one additive selected from the group formed by plasticizers, coalescing agents, colorants, pigments, nacres, lakes, anti-UV agents, preserving agents, surfactants, coating agents, fragrances and moisturizers.

18. Use of an associative polyurethane in a nail varnish with a viscosity of at least 2 Pa.s., measured at 23°C and at a rotation speed of 100 rpm, comprising an aqueous dispersion of film-forming polymer particles.

19. Use of an associative polymer in a nail varnish composition comprising an aqueous dispersion of film-forming polymer particles, in an effective quantity such that the composition has a viscosity of at least 2 Pa.s., measured at 23°C at a rotation rate of 100 rpm.

20. Use of a composition according to any one of Claims 1 to 17, to produce a glossy and/or homogeneous film and/or a film which gives good coverage.

21. Use of a composition according to any one of Claims 1 to 17, as a one-coat varnish.

22. Use of a composition according to any one of Claims 1 to 17, as a film-forming varnish.

23. Method for applying make-up to or for the cosmetic treatment of the nails, **characterized in that** a composition according to any one of Claims 1 to 17 is applied to the nails.

24. Method according to Claim 23, **characterized in that** one coat of the composition is applied.
